**Europäisches Patentamt**

**European Patent Office**

**Office européen des brevets**

(19)

(11) Veröffentlichungsnummer: **0 340 413**

**A1**

(12) # EUROPÄISCHE PATENTANMELDUNG

(21) Anmeldenummer: **89104026.3**

(22) Anmeldetag: **07.03.89**

(51) Int. Cl.⁴: **A61B 17/58**

(30) Priorität: **02.05.88 DE 3814815**

(43) Veröffentlichungstag der Anmeldung:
**08.11.89 Patentblatt 89/45**

(84) Benannte Vertragsstaaten:
**AT BE CH DE FR GB IT LI NL SE**

(71) Anmelder: **fischerwerke Artur Fischer GmbH & Co. KG**
**Weinhalde 14 - 18**
**D-7244 Waldachtal 3/Tumlingen(DE)**

(72) Erfinder: **Fischer, Arthur, Prof. Dr. h.c.**
**Weinhalde 34**
**D-7244 Waldachtal 3/Tumlingen(DE)**

(54) **Spreizdübel für medizinische Anwendungen.**

(57) Es sind Dübel für die Fixation von Knochenfragmenten bekannt, in die eine Befestigungsschraube einsetzbar ist.

Um auch für längere Spreizdübel aussreichend hohe Haltekräfte beim Eindrehen der Befestigungsschraube (16) zu erhalten, besitzt der Spreizdübel mehrere, hintereinander liegende Spreizbereiche (1-4), die um 90 Grad zueinander versetzt angeordnet sind.

Fig. 3

EP 0 340 413 A1

## Spreizdübel für medizinische Anwendungen

Die Erfindung betrifft einen Spreizdübel für medizinische Anwendungen gemäß der Gattung des Hauptanspruchs.

Es sind Spreizdübel für medizinische Anwendungen bekannt, die aus einem gewebeverträglichen Kunststoffmaterial bestehen. Derartige Spreizdübel werden insbesondere zur Fixierung von Knochenfragmenten verwendet. Je nach Art der Knochenfraktur können die Spreizdübel in Verbindung mit Befestigungsschrauben unmittelbar oder unter Verwendung von Abstützplatten eingesetzt werden.

Aus dem DE-GM 25 04 378 ist ein derartiges Befestigungselement für die Osteosynthese bekannt, bei dem die verwendete Dübelhülse ungeschlitzt ist. Durch die Gewindespitzen der in den Dübel eingesetzten Befestigungsschraube wird die Außenwandung der Dübelhülse mit mehreren Wulsten ausgebeult, so daß eine Festsetzung der Dübelhülse erreicht wird. Um ein Aufspreizen zu erhalten, ist es bekannt, Spreizdübel mit Längsschlitzen zu versehen. Im Bereich der Längsschlitze bewirkt der von der Befestigungsschraube ausgeübte Druck auf die Innenwandung des Dübels, daß dieser im Bereich der Längsschlitze aufspreizt. Werden die Längsschlitze am Ende des Dübels angebracht, so läßt sich der Dübel später nur noch sehr schwer aus dem Gewebe entfernen, da das Gewebe mit dem aufgespreizten Dübelende verwächst. Weiterhin haben die mit einer einzigen Spreizzone versehenen Spreizdübel den Nachteil, daß oftmals die Spreizzone in einem Gewebebereich zu liegen kommt, wo aufgrund der Nachgiebigkeit des Gewebes die erforderlichen Haltewerte nicht erreicht werden können.

Der Erfindung liegt die Aufgabe zugrunde, einen Spreizdübel für medizinische Anwendungen zu schaffen, mit dem hohe Haltekräfte erreicht werden können und der auch bei großer Länge beim Eindrehen der Befestigungsschraube im Gewebe gut gegen Verdrehen gesichert ist.

Die Lösung dieser Aufgabe wird bei einem Spreizdübel der eingangs genannten Gattung durch die im Hauptanspruch angegebenen Merkmale erhalten . Der Spreizdübel besitzt mehrere, hintereinander liegende Spreizbereiche, deren Schlitze zueinander um 90 Grad versetzt sind. Dies hat den Vorteil, daß beim Eindrehen der Befestigungsschraube der erste Spreizbereich eine Sicherung gegen Verdrehen durch eine hier auftretende Ausbuchtung des Spreizdübels darstellt. Wird die Befestigungsschraube weiter in den Spreizdübel eingedreht, so weitet sich auch der nachfolgende Spreizbereich auf, wobei jedoch dessen Ausbuchtung um 90 Grad versetzt zur Ausbuchtung des ersten Spreizbereichs ausgerichtet ist. Die versetzte Anordnung der Ausbuchtungen, die auch als Aufspreizungen bezeichnet werden können, erhöht die Wahrscheinlichkeit, daß der Spreizdübel guten Halt im Gewebe findet. Außerdem wirken die hintereinander angeordneten Spreizbereiche, daß mit zunehmendem Eindringen der Befestigungsschraube entsprechend höhere Haltekräfte erzielt werden, so daß dem mit der Eindringtiefe der Befestigungsschraube zunehmenden Drehmoment entsprechend größere Haltekräfte entgegenwirken und somit ein Verdrehen des Spreizdübels auch bei großer Dübellänge vermieden wird.

Damit das Material des Spreizdübels an den Enden der Längsschlitze nicht ausbricht, sind dort Bohrungen oder dgl. vorgesehen.

Insbesondere bei großen Dübellängen ist es vorteilhaft, zwischen den Spreizbereichen ungeschlitzte Ringabschnitte vorzusehen, die als Zentrierungen für die Befestigungsschraube dienen. Ein seitliches Ausbrechen der Befestigungsschraube wird dadurch sicher vermieden. Die Breite dieser ungeschlitzten Ringabschnitte kann den jeweiligen Anforderungen entsprechend gewählt werden, wobei eine geringere Breite auch ein geringeres Drehmoment beim Eindrehen der Befestigungsschraube erfordert.

Für besondere Anwendungsfälle kann es auch zweckmäßig sein, daß die Schlitze der benachbarten Spreizbereiche einander geringfügig überlappen. Dadurch wird insbesondere das zum Eindrehen der Befestigungsschraube erforderliche Drehmoment stark reduziert.

An der Dübelöffnung kann unmittelbar ein geschlitzter Spreizbereich angrenzen, der insbesondere als Sicherung gegen Verdrehen beim Einsetzen der Befestigungsschraube dient. Im Bereich der Dübelöffnung kann ein umlaufender Kragen überstehen, der als Anschlag beim Einsetzen des Dübels in eine Bohrung dient. Soll der Dübel als Durchsteckdübel verwendbar sein, so muß der Dübel ohne überstehenden Kragen ausgebildet sein.

Der Spreizdübel besitzt an seiner Umfangsfläche vorzugsweise ein Rundgewinde, damit der Dübel mit einem geeignetem Drehwerkzeug in eine Gewebebohrung einschraubbar ist. Zu diesem Zweck kann die Innenkontur des Spreizdübels als Innensechskant ausgebildet sein, um einen Sechskant-Schraubenschlüssel einsetzen zu können. Der Spreizdübel läßt sich somit auch später unter Verwendung des Drehwerkzeuges wieder aus dem Gewebe ausschrauben.

Die Erfindung wird nachfolgend anhand der Zeichnung näher erläutert. Es zeigen:

Figur 1 einen Spreizdübel mit mehreren Spreizbereichen,

Figur 2 einen Spreizdübel mit überstehendem Kragen an der Dübelöffnung und

Figur 3 ein Anwendungsbeispiel eines im Knochen verankerten Spreizdübels.

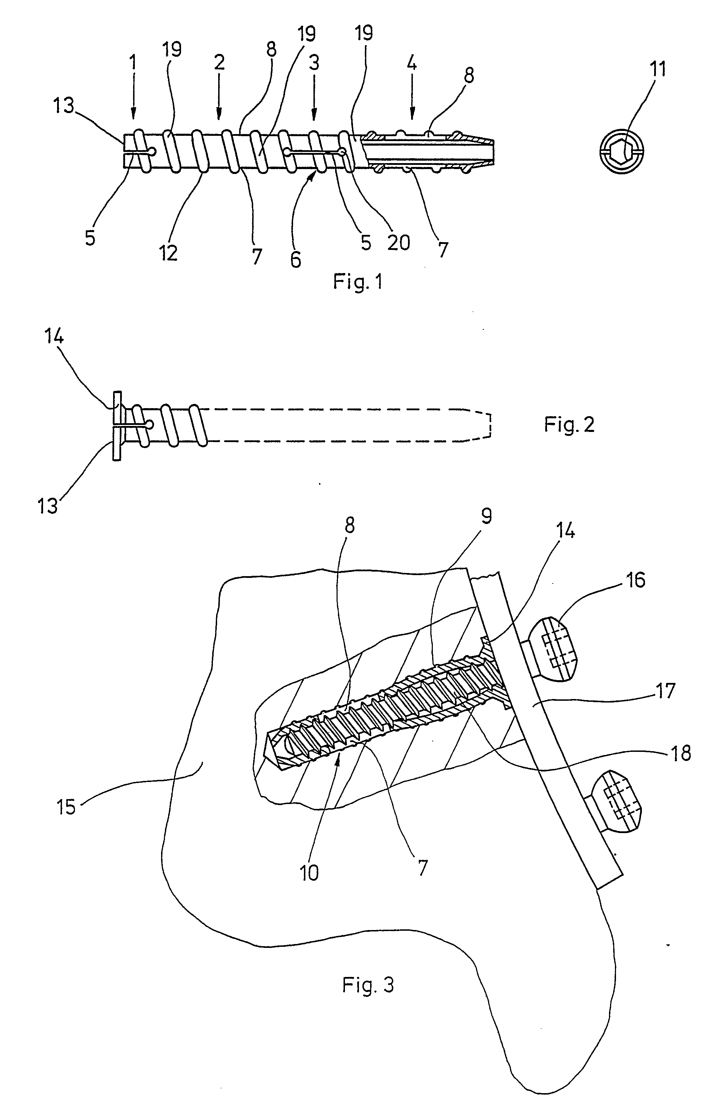

Der in Figur 1 dargestellte Spreizdübel besitzt mehrere Spreizbereiche 1 bis 4, die jeweils zwei gegenüberliegende Längsschlitze 5, 6 bzw. 7, 8 aufweisen. Die Längsschlitze 5, 6 sind gegenüber den Längsschlitzen 7, 8 um 90 Grad versetzt. Wird nun in den Spreizdübel eine Befestigungsschraube eingesetzt, wie dies in Figur 3 dargestellt ist, so treten in entsprechender Weise um 90 Grad versetzt Ausbuchtungen 9, 10 auf. Die Ausbuchtung 10 ist in Figur 3 nicht sichtbar, da sie um 90 Grad zur Ausbuchtung 9 versetzt ist.

Der in Figur 1 dargestellte Spreizdübel besitzt eine als Innensechskant 11 ausgebildete Innenkontur, in die ein ensprechend geformter Sechskant-Drehschlüssel als Drehwerkzeug einsetzbar ist. Der Spreizdübel kann somit mittels eines geeigneten Drehwerkzeugs und aufgrund seines über seine gesamte Länge sich erstreckenden Rundgewindes 12 in eine Bohrung eines Gewebes eingeschraubt und wieder ausgeschraubt werden.

In Figur 2 ist eine Ausführungsform eines Spreizdübels gemäß Figur 1 angedeutet, bei dem jedoch im Bereich der Dübelöffnung 13 ein überstehender Kragen 14 als Anschlag ausgebildet ist.

In Figur 3 ist ein Anwendungsbeispiel dargestellt, bei dem der Spreizdübel in einen Knochen 15 eingesetzt ist. Eine Befestigungsschraube 16 greift durch eine Abstützplatte 17 mit ihrem Gewindeabschnitt 18 in den Spreizdübel ein, wodurch dieser in zwei Spreizbereichen aufspreizt und Ausbuchtungen 9, 10 ausbildet.

Der Erfindungsgemäße Spreizdübel kann insbesondere wesentlich länger als in Figur 3 dargestellt ausgebildet sein, wobei durchaus mehr als vier hintereinander angeordnete Spreizbereiche vorgesehen sein können.

Um insbesondere bei langen Spreizdübeln eine gute Zentrierung der Befestigungsschraube beim Eindrehen zu erhalten, können zwischen den Spreizbereichen ungeschlitzte Ringabschnitte 19 vorgesehen sein, wie sie in Figur 1 dargestellt sind. Damit beim Aufspreizen der Spreizbereiche an den Enden der Längsschlitze keine Risse entstehen, sind dort Bohrungen 20 vorgesehen.

## Ansprüche

1. Spreizdübel für medizinische Anwendungen, insbesondere für die Fixation von Knochenfragmenten mittels einer Abstützplatte, der wenigstens einen bei Eindrehen einer Befestigungsschraube aufspreizbaren, längsgeschlitzten Spreizbereich hat, **dadurch gekennzeichnet, daß** über die gesamte Länge des Spreizdübels mehrere, hintereinander liegende Spreizbereiche (1 bis 4) vorgesehen sind, und daß die Schlitze (5, 6; 7,8) benachbarter Spreizbereiche (3; 4) um 90 Grad zueinander versetzt sind.

2. Spreizdübel nach Anspruch 1, **dadurch gekennzeichnet,** daß die Enden der Schlitze (5 bis 8) von Bohrungen oder dgl. ausgebildeten Ausbuchtungen gebildet sind.

3. Spreizdübel nach einem der Ansprüche 1 oder 2, **dadurch gekennzeichnet,** daß zwischen den Spreizbereichen, (1 bis 4) ungeschlitzte Ringabschnitte (19) ausgebildet sind.

4. Spreizdübel nach einem der Ansprüche 1 oder 2, **dadurch gekennzeichnet,** daß sich die Schlitze (5 bis 8) der benachbarten Spreizbereiche (3,4) überlappen.

5. Spreizdübel nach einem der vorhergehenden Ansprüche, **dadurch gekennzeichnet,** daß von der Dübelöffnung (13) gegenüberliegende Längsschlitze ausgehen, die einen verkürzten ersten Spreizbereich (1) bilden.

6. Spreizdübel nach einem der vorhergehenden Ansprüche, **dadurch gekennzeichnet,** daß an der Dübelöffnung (13) ein überstehender Kragen (14) ausgebildet ist.

7. Spreizdübel nach einem der vorhergehenden Ansprüche, **dadurch gekennzeichnet,** daß die Innenkontur des Spreizdübels als Innensechskant (11) ausgebildet ist.

Fig. 1

Fig. 2

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 209 685 (FISCHER-WERKE ARTUR FISCHER) <br> * Ansprüche 1-2,9; Figur 1 * <br> --- | 1-7 | A 61 B 17/58 |
| Y | DE-A-3 602 294 (AUDI) <br> * Ansprüche 4-5,7-9,11; Figuren 1-4 * <br> --- | 1-7 | |
| A | DE-U-8 710 681 (F. ZELGER) <br> * Ansprüche 1,3; Figur 8 * <br> --- | 1,3 | |
| A | DE-A-2 314 520 (A. BERNER) <br> * Figuren 1-3 * <br> --- | 1,4 | |
| A,D | DE-U-8 504 378 (A. FISCHER) <br> * Ansprüche 1,3-4; Figuren 1-2 * <br> ----- | 1,6 | |

RECHERCHIERTE SACHGEBIETE (Int. Cl.4)

A 61 B
F 16 B

Der vorliegende Recherchenbericht wurde für alle Patentansprüche erstellt

| Recherchenort | Abschlußdatum der Recherche | Prüfer |
|---|---|---|
| DEN HAAG | 04-08-1989 | NICE P.R. |